# EUROPEAN PATENT SPECIFICATION

(11) **EP 2 568 960 B1**
(45) Date of publication and mention of the grant of the patent: **06.06.2018**
(21) Application number: 11780312.2
(22) Date of filing: 04.05.2011
(51) Int. Cl.: A61K 9/00, A61K 47/12, A61K 47/18, A61K 39/395

(54) **LIQUID FORMULATION OF POLYPEPTIDES CONTAINING AN FC DOMAIN OF AN IMMUNOGLOBULIN**
FLÜSSIGE FORMULIERUNG AUS POLYPEPTIDEN MIT EINER FC-DOMÄNE EINES IMMUNGLOBULINS
PRÉPARATION LIQUIDE DE POLYPEPTIDES CONTENANT UN DOMAINE Fc D'UNE IMMUNOGLOBULINE

(30) Priority: 10.05.2010 IN 1465MU2010
(43) Date of publication of application: 20.03.2013
(73) Proprietor: Intas Pharmaceuticals Ltd., Ahmedabad, Gujarat 380009 (IN)
(72) Inventor: TUNGA, Binita Shrivastava, Ahmedabad 380 015, Gujarat (IN); SHARMA, Sachin, Delhi 110 041 (IN); DUA, Rajiv, Ahmednagar 414 002 (IN); DUTTA, Sourabh, New Delhi 110 048 (IN); MALLUBHOTLA, Hanuman, Ahmedabad 380 015, Gujarat (IN); PANDEY, Vijaykant, Vejalpur, Ahmedabad 380051 Gujarat (IN); CHHATBAR, Chandresh, Memnagar, Ahmedabad 380052, Gujarat (IN)
(74) Representative: Accord Healthcare, S.L.U.
(86) International application number: PCT/IN2011/000313
(87) International publication number: WO 2011/141926

(56) References cited:
- WO-A2-2007/092772
- WO-A2-2010/077422
- US-A1- 2006 240 520
- US-A1- 2008 050 370
- US-A1- 2008 071 063

## Description

### FIELD OF THE INVENTION

The present invention relates to an aqueous formulation comprising TNFR:Fc, methods of manufacture of the composition, methods of administration and kits containing the same.

### BACKGROUND OF THE INVENTION

Tumour necrosis factor is a polypeptide cytokine involved in inflammation and the acute phase response. TNF-alpha is present in larger quantities in persons with rheumatoid arthritis or Crohn's disease. Direct inhibition of TNF-alpha by the biological agents has produced significant advances in rheumatoid arthritis treatment and has validated the extra-cellular inhibition of this pro-inflammatory cytokine as an effective therapy. One such biological agent is Etanercept.

Etanercept (TNFR:Fc) is a dimeric fusion protein consisting of the extra-cellular ligand-binding portion of the human 75 kilo Dalton (p75) tumor necrosis factor receptor (TNFR) linked to the Fc portion of human IgG1. The Fc component of Etanercept consists of the CH² domain, the CH³ domain and hinge region, whereas the CH¹ domain is absent. It is produced through recombinant DNA technology in a Chinese hamster ovary mammalian cell expression system. It consists of 934 amino acids, and has an apparent molecular weight of approximately 150 kilo Dalton.

Generally, proteins have a very short half-life, and undergo denaturation (such as aggregation, dissociation, and adsorption on the surface of vessels) upon exposure to various factors such as unfavorable temperatures, water-air interface, high-pressure, physical/ mechanical stress, organic solvents and microbial contamination. Consequently, the denatured protein loses intrinsic physicochemical properties and physiological activity. Denaturation of proteins is often irreversible, and therefore proteins, once denatured, may not recover their native properties to the initial state.

In the biopharmaceutical industry, the long term storage of proteins, prepared using recombinant DNA technology in aqueous formulations, is generally a difficult task. To overcome the stability problem of proteins in aqueous formulations, therapeutic protein products are made more stable via lyophilization (freeze-drying). Lyophilized products are usually accompanied by sterile aqueous media for reconstitution. After reconstitution, the formulations typically have short useful storage lives, even when stored at low temperatures (e.g., 5°C). Example of TNF alpha inhibitors which are available in the market in the lyophilized form are Enbrel® and Remicade® and both the compositions should be reconstituted before use.

Typical practices to improve polypeptide stability can be addressed by varying the concentration of elements with the formulation, or by adding excipients to modify the formulation.

US5580856 discloses the stabilization of dried proteins against loss of biological activity in the formulations by adding a reconstitution stabilizer upon rehydration of the dried protein. A kit for producing a formulation by dissolving the dried composition in a solvent containing the reconstitution stabilizer is also described.

US6171586 discloses a stable aqueous pharmaceutical formulation comprising a therapeutically effective amount of an antibody not subjected to prior lyophilization, a buffer maintaining the pH in the range from about 4.5 to about 6.0, a surfactant and a polyol, along with uses for such a formulation.

EP1314437 relates to an invention of stabilized preparations containing an antibody in a glycine buffer and/ or a histidine buffer and also provides processes for preparing a protein-containing stabilized preparation, comprising adjusting the pH with a basic amino acid or a basic amino acid derivative or a salt thereof.

EP1478394 discloses about the invention that relates to an aqueous pharmaceutical composition suitable for long-term storage of polypeptides containing an Fc domain of an immunoglobulin, methods of manufacture, methods of administration and kits containing same.

WO 2007/092772 discloses liquid formulation of Fc variant protein that improves the stability in part by reducing the propensity of such molecules to rapidly aggregate.

WO 2010/077422 relates to formulations of single domain antigen binding molecule, e.g, nanobody molecules, in particular formulation of TNF-binding nanobody molecules.

US20060240520 discloses nucleic acids encoding BAFF-R polypeptides, as well as antibodies to BAFF-R polypeptides and pharmaceutical compositions including same.

US20080071063 provides formulations of proteins comprising a variant Fc region that improves the stability in part by reducing the propensity of such molecules to rapidly aggregate.

Thus, it is desirable to provide liquid formulations of TNFR: Fc having enhanced stability under refrigeration and at least moderate stability at normal room temperatures, and to avoid the inconvenience and potential for errors from the reconstitution procedure.

### SUMMARY OF THE INVENTION

The invention provides a novel liquid formulation comprising TNFR:Fc which exhibits long term stability at 4°C and room temperatures.

In one embodiment, the invention provides an aqueous pharmaceutical formulation comprising TNFR:Fc, an aggregation inhibitor, a buffer, a non-ionic surfactant, a stabilizer, a tonicity modifier, and optionally a preservative, wherein the aggregation inhibitor is aspartic acid, optionally in combination with lysine and wherein the pharmaceutical formulation further comprises EDTA as chelating agent and fragmentation inhibitor.

In another embodiment of the invention, the buffer of the formulation comprises phosphate buffer, bicarbonate buffer, succinate buffer, acetate buffer, citrate buffer, amino acids, TRIS buffer, either alone or in suitable combination, giving desired pH range from 5.5 to 7.5, and preferably the buffer is selected from the group consisting of sodium phosphate, histidine, potassium phosphate, sodium or potassium citrate, maleic acid, ammonium acetate, tris-(hydroxymethyl)- aminomethane (tris), acetate and diethanolamine or a combination thereof, and more preferably the buffer is phosphate buffer in combination with its sodium and potassium salts.

In yet another embodiment of the invention, the non-ionic surfactant of the formulation is selected from the group consisting of a polysorbate-based non-ionic surfactant and a poloxamer-based non-ionic surfactant or a combination thereof, and preferably the non-ionic surfactant is polysorbate 20.

In yet another embodiment of the invention, the stabilizer of the formulation is selected from the group consisting of amino acids such as glycine, alanine, lysine, proline and serine and their salts thereof, either alone or in combination, monosaccharides such as glucose and mannose, and their likes, either alone or in combination, disaccharides such as sucrose, trehalose, and maltose, and their likes, either alone or in combination, sugar alcohols such as mannitol and sorbitol, and their likes, either alone or in combination, and polysaccharides such as dextran, polyethylene glycol and their likes, either alone or in combination, and preferably the stabilizer is sucrose.

In yet another embodiment of the invention, the tonicity modifier of the formulation is selected from the group consisting of sodium chloride, potassium chloride, sodium sulfate or a combination thereof, and preferably the tonicity modifier is sodium chloride.

In yet another embodiment of the invention, the preservative of the formulation is selected from the group consisting of phenol, m-cresol, p-cresol, o-cresol, chloro-cresol, alkylparaben (methyl, ethyl, propyl, butyl and the like), benzethonium chloride, sodium dehydroacetate or thiomersal, either alone or in combination thereof.

In a preferred embodiment of the invention, the formulation comprises aspartic acid and lysine as aggregation inhibitors, sodium-potassium phosphate buffer giving desired pH range from 5.5 to 7.5, sodium chloride as tonicity modifier, sucrose as stabilizer, polysorbate 20 as a non-ionic surfactant, EDTA as fragmentation inhibitor and chelating agent and TNFR:Fc at a concentration of 10 mg/ml to 100 mg/ml.

In a more preferred embodiment of the invention, the formulation comprises from 25 to 50 mg/ml of TNFR:Fc, from 1 mM to 50 mM of aspartic acid, from 1 mM to 50 mM of lysine, from 10 mM to 50 mM of sodium-potassium phosphate, from 0.75 % to 3 % of sucrose, from 50 to 150 mM ofNaCl, from 0.1 mM to 2 mM of EDTA, from 0.05 mg/ml to 1.0 mg/ml of polysorbate 20, and at pH 6.0 to pH 7.0.

In an even more preferred embodiment of the invention, the formulation is an injectable aqueous solution.

In another aspect, the invention discloses the use of EDTA for preventing fragmentation of TNFR:Fc in an aqueous pharmaceutical formulation.

In yet another aspect, the present invention provides for a polypeptide containing an Fc domain of an immunoglobulin selected from the group consisting of monoclonal antibody, fusion protein and TNFR:Fc.

### BRIEF DESCRIPTION OF THE DRAWINGS

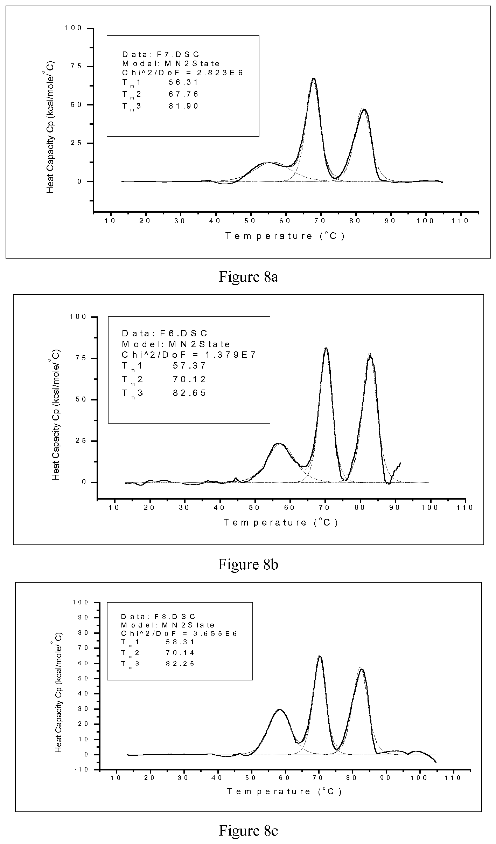

**Figure 1a** **(Non-reducing) &** **Figure 1b** **(Reducing)** shows the comparison of degradation pattern of Etanercept formulations with different aggregation inhibitors kept at 40°C on zero day.
   **Lane 1:** Reference Medicinal Product
   **Lane 2:** F-1 as given in Table 1
   **Lane 3:** F-2 as given in Table 1
   **Lane 4:** F-3 as given in Table 1
   **Lane 5:** F-4 as given in Table 1
**Figure 2a** **(Non-reducing) &** **Figure 2b** **(Reducing)** shows the comparison of degradation pattern of Etanercept formulations with different aggregation inhibitors kept at 40°C for 1 month.
   **Lane 1:** Reference Medicinal Product
   **Lane 2:** F-1 as given in Table 1
   **Lane 3:** F-2 as given in Table 1
   **Lane 4:** F-3 as given in Table 1
   **Lane 5:** F-4 as given in Table 1
**Figure 3a** **(Non-reducing) &** **Figure 3b** **(Reducing)** shows the comparison of degradation pattern of Etanercept formulations with different aggregation inhibitors kept at 47°C for 16 hours.
   **Lane 1:** Reference Medicinal Product
   **Lane 2:** F-3 as given in Table 1
   **Lane 3:** F-1 as given in Table 1
   **Lane 4:** Marker
**Figure 4** shows the comparison of degradation pattern of Etanercept formulations with different aggregation inhibitors kept at 55°C for 24 hours.
   **Lane 1:** Reference Medicinal Product
   **Lane 2:** F-3 as given in Table 1
   **Lane 3:** F-1 as given in Table 1
   **Lane 4:** Marker
**Figure 5a** **(Non-reducing) &** **Figure 5b** **(Reducing)** shows the comparison of degradation pattern of Etanercept formulations with lysine as stabilizer, kept at 40°C for 7 days.
   **Lane 1:** Reference Medicinal Product
   **Lane 2:** F-5 as given in Table 1
   **Lane 3:** F-3 as given in Table 1
   **Lane 4:** Marker
**Figure 6a** **(Non-reducing) &** **Figure 6b** **(Reducing)** shows the comparison of degradation pattern of Etanercept formulations kept at 40°C on Zero day for checking the effect of EDTA.
   **Lane 1:** Without EDTA
   **Lane 2:** With EDTA
   **Lane 3:** Marker
**Figure 7a** **(Non-reducing) &** **Figure 7b** **(Reducing)** shows the comparison of degradation pattern of Etanercept formulations kept at 40°C for 3 days.
   **Lane 1:** Without EDTA
   **Lane 2:** With EDTA
   **Lane 3:** Marker
**Figure 8a, Figure 8b and Figure 8c** shows the DSC profile of Etanercept formulations with different buffer and their salt.
**Figure 9** shows comparative transition midpoints of formulations (F6 to F8).
**Figure 10** shows comparative bioactivity of Final formulated bulk and RMP
**Figure 11** shows comparative degradation by SE=HPLC in F6, F8 and RMP.
**Figure 12** shows comparative HIC profile of F8 and RMP kept at -20°C for 3 months
**Figure 13** shows comparative HIC profile of F8 and RMP kept at 5±3°C °C for 3 months
**Figure 14** shows comparative HIC profile of F8 and RMP kept at 25±2°C °C for 3 months
**Figure 15** shows comparative HIC profile of F8 and RMP kept at 40°C for 3 months

### DESCRIPTION OF THE INVENTION

The present pharmaceutical formulation comprises a purified polypeptide, buffer, aggregation inhibitors, tonicity modifiers, stabilizers, surfactants, chelating agents and optionally with preservative in suitable combinations thereof.

Monoclonal antibodies are used in high concentration for therapeutic use and the high concentration of protein is known to increase aggregation. In one aspect, aggregation inhibitors are required in formulation compositions to keep the product in native state as well as biologically active with enhanced shelf life and storage conditions. There are a few amino acids which act as aggregation inhibitors by increasing surface tension, preferential binding or hydration and preferential interaction.

Aggregation inhibitors reduce a polypeptide's tendency to form aggregates. The amino acids like aspartic acid, phenylalanine, glutamic acid, alanine, histidine and lysine act to reduce aggregation of Fc domain of TNFR:Fc in a formulation for long periods and are preferred embodiments of the invention.

In another aspect, buffers are required for maintaining pH of the formulation. The buffer system of the present invention comprises phosphate buffer, bicarbonate buffer, succinate buffer, acetate buffer, citrate buffer, amino acids, TRIS buffer, either alone or in suitable combination, giving desired pH range from 5.5 to 7.5.

In the present invention, surfactant is used in order to prevent adsorption of TNFR:Fc on the surface of the vial, ampoule, carpoule, cartridge or syringe. Surfactants lower surface tension of a protein solution, thereby, preventing its adsorption or aggregation on to a hydrophobic surface. Preferred surfactants of the present invention include a polysorbate-based non-ionic surfactant and polyoxyethylene copolymer, polyvinylpyrrolidone, either alone or in combination.

In one embodiment, stabilizers used in the present invention are selected from the group that consists of: amino acids such as glycine, alanine, lysine, praline, serine and the like and its salts thereof, either alone or in combination, monosaccharide such as glucose and mannose, and their likes, either alone or in combination, disaccharides such as sucrose, trehalose, and maltose, and their likes, either alone or in combination, sugar alcohols such as mannitol and sorbitol, and their likes, either alone or in combination, and polysaccharides such as dextran , polyethylene glycol and their likes, either alone or in combination.

A tonicity modifier is understood to be a molecule that contributes to the osmolality of a solution. The osmolality of a pharmaceutical composition is preferably regulated in order to maximize the active ingredient's stability and also to minimize discomfort to the patient upon administration. Examples of tonicity modifiers suitable for modifying osmolality include, but are not limited to amino acids (arginine, cysteine, histidine and the like), salts (sodium chloride, potassium chloride, calcium chloride and the like), and/or saccharides (sucrose, glucose, mannitol and their likes).

Etanercept is prone to fragmentation during storage, so some fragmentation inhibitor is required for making optimum formulation composition for etanercept. EDTA known to have an impact in preventing fragmentation in many of the products such as Interferon, Alemtuzumab, etc is used in the present formulation.

Chelating agents stabilize or prevent the free metal ions to react with protein of interest. Examples of chelating agents that can be used in the present invention includes but not limited to EDTA (ethylenediamine tetraacetic acid), HEDTA (hydroxyethylenediamine triacetic acid), NTA (nitriolotriacetic acid), DTPA (Diethylenetriamine pentaacetate) and citric acid.

Preservative refers to a composition or substance added to a formulation to act as a bacteriostatic agent. A preserved TNFR: Fc containing formulation of the present invention preferably meets statutory or regulatory guidelines for preservative effectiveness to be a commercially viable multi-use product, preferably in humans. Preservatives used in the present invention are selected from the group consisting of phenol, m-cresol, p-cresol, o-cresol, chloro-cresol, alkylparaben (methyl, ethyl, propyl, butyl and the like), benzethonium chloride, sodium dehydroacetate or thiomersal, either alone or in combination thereof. However, in the present invention, the use of preservative is optional and is preferred while designing multi-dose formulations.

The novel, thermostable, aqueous formulation of TNFR: Fc described in the present invention has the following advantages;
1. Involves use of aggregation inhibitor, which prevents the aggregation of the fusion protein during long term storage.
2. Involves use of a stabilizer which prevents unwanted cleavages during long term storage.
3. Provides better stability to the aqueous formulation to maintain its activity for a prolonged period of time to guarantee a reasonable shelf-life.
4. Provides better stability to the aqueous formulation even at elevated temperatures.

The following examples illustrate the pharmaceutical compositions described in the present invention and the means of carrying out the invention to obtain a stable aqueous pharmaceutical formulation of TNFR: Fc. The examples are in no way to be construed as limitations for the scope of the invention.

### Example 1

### Screening and selection of aggregation inhibitors

### Screening of aggregation inhibitors at 40°C

Formulation of Etanercept with different aggregation inhibitors like lysine, aspartic acid, glycine and proline were studied. These formulations also contained other excipients as per details given in Table 1. Samples and RMP were incubated at 40°C for one month and then analyzed by SDS-PAGE. SDS-PAGE was used as an analytical technique which can separate the dissociated and high molecular weight species from native protein based on molecular weight. Due to high propensity to aggregate in case of high concentration of monoclonal antibodies and fusion proteins, non-reducing SDS PAGE was utilized to assess covalent aggregates. As there are many disulphide bonds present in fusion proteins as well as monoclonal antibodies, the fragmentation of proteins was checked under reducing SDS PAGE.

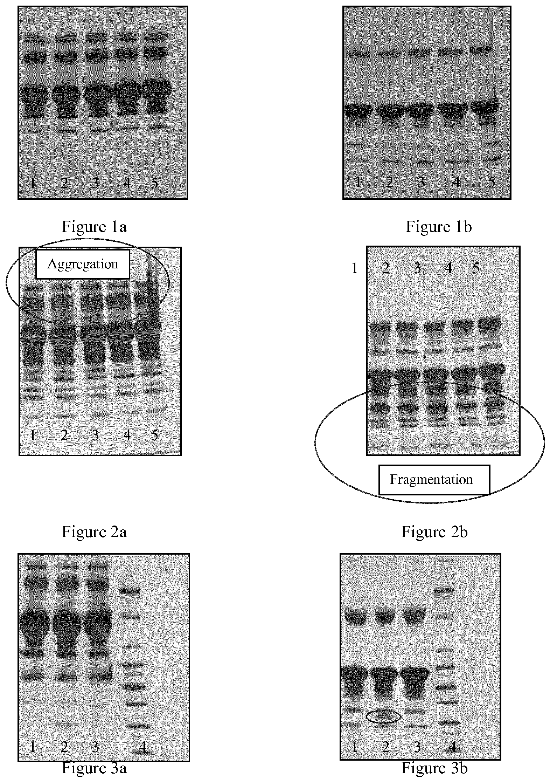

SDS-PAGE gel (Fig. 1a, 1b, 2a & 2b) of different formulations shows the aggregation and fragmentation of Etanercept after one month at 40°C.

**Table 1: Identification of aggregation inhibitor**

| **Sr. No** | **Components** | **Formulation No.** | | | |
|---|---|---|---|---|---|
| | | **F1** | **F2** | **F3** | **F4** |
| 1. | Etanercept | 50 mg | 50 mg | 50 mg | 50 mg |
| 2. | Sucrose | 20 mg | 20 mg | 20 mg | 20 mg |
| 3. | Phosphate Buffer | 25 mM | 25 mM | 25 mM | 25 mM |
| 4. | DL-Aspartic acid | 20 mM | - | - | - |
| 5. | L-Lysine Monohydrate | - | 20 mM | - | - |
| 6. | Glycine | - | - | 20 mM | - |
| 7. | Proline | - | - | - | 20 mM |
| 8. | Sodium Chloride | 100 mM | 100 mM | 100 mM | 100 mM |
| 9. | Polysorbate 20 | 0.2 mg | 0.2 mg | 0.2 mg | 0.2 mg |
| 10. | Disodium EDTA | 1 mM | 1 mM | 1 mM | 1 mM |

F2 and F4 were found to have more degradation in terms of aggregation as well as fragmentation but there is no major difference in terms of aggregates and fragments observed for F1 and F3. As no significant difference in impurities was observed at this temperature, both (F1 and F3) the formulations were further studied at higher temperatures to increase degradation kinetics as well as the degradants.

### Screening of aggregation inhibitors at 47°C and 55°C

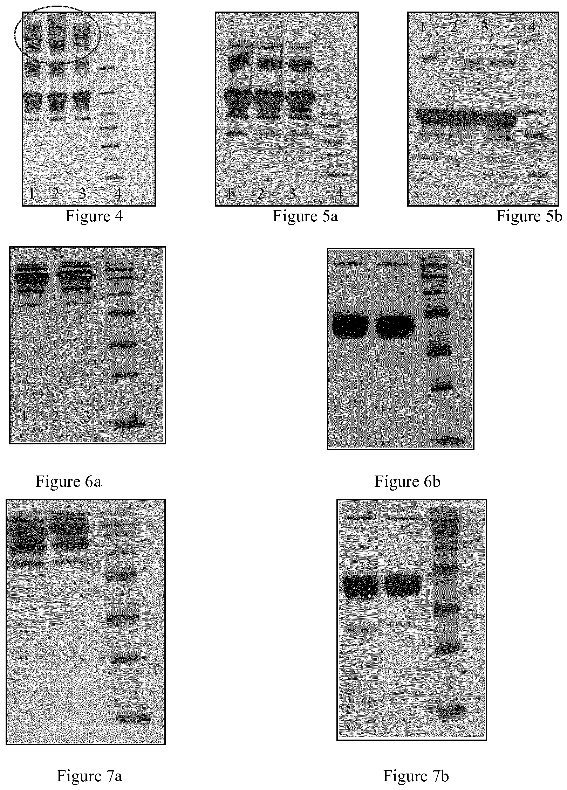

To find out the best aggregation inhibitor between aspartic acid (F1) and glycine (F3), both the formulations were charged at 47°C as well as at 55°C. The results indicate that Glycine (F3) is showing higher degradation in terms of aggregation as well as fragmentation as compared to aspartic acid (F1) at both the temperatures tested i.e. 47°C for 16 hours (Fig. 3a & 3b) and 55°C for 24 hours (Fig. 4). Formulation containing glycine is also showing one extra fragment band (Fig. 3b)) in reducing condition at 47°C; 16 hours.

Aspartic acid is shown to be effective against aggregation as well as fragmentation at all the temperatures without any extra band compared to reference medicinal product (RMP). An extra band observed in formulation containing glycine could be contributor to immunogenicity.

### Combination of Excipients (Lysine + Aspartic acid)

In order to maintain pH of F1 formulation containing Aspartic acid (shown to be effective in all temperatures as seen above), lysine buffer was added. Both Lysine and aspartic acid were used at a concentration of 10 mM as these amino acids are expected to have higher stability at equimolar concentrations. The formulation composition is mentioned in Table 1a:

**Table 1a: Formulation of F5**

| **Sr. No** | **Components** | **Composition F5** |
|---|---|---|
| 1. | Etanercept | 50 mg |
| 2. | Sucrose | 20 mg |
| 3. | Phosphate Buffer | 25 mM |
| 4. | Aspartic acid | 10 mM |
| 5. | Lysine Monohydrate | 10 mM |
| 6. | Sodium Chloride | 100 mM |
| 7. | Polysorbate 20 | 0.2 mg |
| 8. | Disodium EDTA | 1 mM |

The formulation containing Lysine in combination with Aspartic acid (F5) showed less fragmentation compared to the formulation without lysine (F3) as seen in Figure 5a and 5b.

### Example 2

### Effect of EDTA

The effect of EDTA was checked in F3 formulation at 40°C for 3 days and analyzed by SE-HPLC as size exclusion chromatography separates proteins and its related impurities on the basis of their size. This technique is useful to detect aggregation and fragmentation of Etanercept and the results are given below in Table 2.
SDS PAGE was also used to compare aggregates and fragments pattern. (Figure 5a, 5b, 6a and 6b)

**Table 2: SE-HPLC Results of formulations showing impact of EDTA**

| **Type** | **Zero Day** | | **40°C for 3 Days** | |
|---|---|---|---|---|
| | **w/o EDTA** | **with EDTA** | **w/o EDTA** | **with EDTA** |
| **Dimer** | 2.20 | 2.40 | 1.62 | 2.18 |
| **Oligomers** | 0.0007 | 0 | 0.005 | 0.0 |
| **Degradants** | 0.003 | 0.39 | 1.39 | 0.82 |
| **Purity** | 97.79 | 97.21 | 96.98 | 97.00 |

F3 was found to have less rate of degradation to that of formulation without EDTA as per SE-HPLC results particularly in terms of fragments.

BY SDS PAGE, F3 is found to have fewer amounts of low molecular weight bands after exposure of samples at 40°C for 3 days. Hence, EDTA plays an important role in preventing fragmentation of Etanercept.

### Example 3

### Effect of buffers

Etanercept formulations with phosphate buffer and histidine buffer as given in Table 3 (F6 and F7) were charged at 50°C for 2 days and analyzed by SE-HPLC and Differential Scanning Calorimetry (DSC). DSC is a technique used to determine thermodynamic stability of proteins. Etanercept has three transitions and Tm1 corresponds to TNFR, Tm2 corresponds to CH2 domain and Tm3 corresponds to CH3 domain of Fc portion (higher Tm indicates higher stability)

**Table 3: Identification of buffer component**

| **Sr. No** | **Components** | **Formulation No.** | |
|---|---|---|---|
| | | **F6** | **F7** |
| 1 | Sucrose | 20 mg | - |
| 2 | Trehalose | - | 20 mg |
| 3 | Lysine Monohydrate | 10 mM | - |
| 4 | Glycine | - | 25 mM |
| 5 | Aspartic acid | 10 mM | 5 mM |
| 6 | Sodium Chloride | 100 mM | 100 mM |
| 7 | Polysorbate 20 | 0.2 mg | 0.2 mg |
| 8 | Disodium EDTA | 1 mM | 1 mM |
| 9 | Phosphate Buffer | 25 mM | - |
| 10 | Histidine | - | 10 mM |

**Table 4: SE-HPLC results for Buffer component identification**

| **Formulation No.** | **% Aggregates** | **% Fragments** | **% Purity** |
|---|---|---|---|
| F6-0 day | 1.4 | 0 | 98.6 |
| F7-0 day | 1.6 | 0.1 | 98.3 |
| F6-2 day | 12.5 | 0 | 87.5 |
| F7-2 day | 21.6 | 0 | 78.4 |

The composition with phosphate buffer as buffering agent was having high purity and lower aggregates compared to formulations containing histidine buffer as per SE-HPLC results.

As per DSC analysis, transition midpoints of first and second transition peak of phosphate buffer are around 1°C and 2°C respectively higher than that of histidine buffer formulation. The midpoint of third transition peak is almost similar in both the buffers.

As phosphate buffer was observed to have less degradation rate compared to histidine buffer as evidenced by SE-HPLC results and high thermodynamic stability evidenced by DSC analysis, phosphate buffer is preferred as suitable buffer system.

### Example 4

### Buffer salt identification

The buffer salts (Sodium and Potassium) were screened for best suitable candidate in terms of stability.
The Phosphate buffer in combination of its salts i.e. sodium / potassium was used for desired stability of protein. Table 5 summarizes the combination sets used for selecting the best formulation candidate.

**Table 5: Identification of combination buffer salt and its concentration**

| **Sr. No** | **Components** | **F6** | **F8** |
|---|---|---|---|
| 1 | Sucrose | 20 mg | 20 mg |
| 2 | Lysine Monohydrate | 10 mM | 10 mM |
| 3 | Aspartic acid | 10 mM | 10 mM |
| 4 | Sodium Chloride | 100 mM | 100 mM |
| 5 | Polysorbate 20 | 0.2 mg | 0.2 mg |
| 6 | Disodium EDTA | 1 mM | 1 mM |
| 7 | Sodium Phosphate monobasic | 11 mM | 11 mM |
| 8 | Sodium phosphate dibasic | 13 mM | - |
| 9 | Potassium phosphate dibasic | - | 19 mM |

The thermodynamic stability of compositions was identified with the means of DSC. The aggregation and fragmentation in due course of storage was assessed by means of SE-HPLC. The results are reported in Table 6 and Table 7.

**Table 6: Experimental DSC results of selected combinations**

| S. No. | Formulations | Tm1 | Tm2 | Tm3 |
|---|---|---|---|---|
| 1 | RMP | 57.5 | 70.4 | 82.3 |
| 2 | F6 | 57.7 | 70.1 | 82.7 |
| 3 | F8 | 58.3 | 70.1 | 82.3 |

**Table 7: SE-HPLC results for buffer salt optimization**

| Study Temperature | Duratio n (days) | Formulations | | |
|---|---|---|---|---|
| | | RMP | F6 | F8 |
| 50°C | 0 | 98.4 | 98.6 | 98.6 |
| | 2 | 81.4 | 86.1 | 87.5 |
| | 3 | 75.9 | Not Done | 80.9 |

The composition with potassium salt was having high purity as compared to formulations containing sodium salt as per the SE-HPLC results.

As per DSC analysis, transition midpoint of first transition peak of potassium salt is around 1°C higher than that of sodium salt composition only. The midpoints of second and third transition peak are almost similar in both the salts.

As sodium-potassium salt combination was observed to have less degradation rate compared to sodium salt alone as evidenced by SE-HPLC results and high thermodynamic stability evidenced by DSC analysis, the sodium-potassium salt combination was chosen as suitable buffer system.

### Example 5

### Preparation of TNFR: Fc fusion protein with aggregation inhibitors L-aspartic acid and Lysine

All the components given in the table 8 except polysorbate 20 were dissolved in 80 % of water and mixed them properly with continuous stirring. Polysorbate 20 was added in the solution by making final concentration as 0.2 mg/ml. The volume of formulation buffer was made to 90 % with water. The pH was adjusted to 6.3 with any suitable acid/base and the volume was made up to 100 % with water. The formulation buffer was filtered and the Etanercept bulk was diluted in required concentration with this filtered formulation buffer. The Etanercept formulation was analyzed by SE-HPLC, SDS-PAGE, and HIC at zero time, and after 1, 2, and 3 months storage at -20°C, 5±3°C, 25±2°C. HIC separates proteins on the basis of hydrophobicity. Peak 1 in HIC determines fragments, Peak 2 corresponds to active dimer of Etanercept and Peak 3 corresponds to aggregates. Pre-peak 1 observed in some cases corresponds to truncated impurities.

The bioactivity of samples was also checked. The method is based on principle of neutralization of cytotoxic effect. TNF-α produces cytotoxic effect on L929 (mouse connective tissue) cell line. TNFR:Fc specifically neutralizes the cytotoxic activity of TNF-α in a dose dependent manner. The bioactivity of RMP is 1.7 Million units per mg. The results are reported in Table 9.

The present pharmaceutical formulation was prepared by adding aggregation inhibitors to the purified polypeptide as described above. Further, a buffer, a tonicity modifier, a surfactant, a fragmentation inhibitor and an additional excipient can be added as needed. It will be understood by one of ordinary skill in the art that the combining of the various components to be included in the composition can be done in any appropriate order, namely, the buffer can be added first, middle or last and the tonicity modifier can also be added first, middle or last. It is also to be understood by one of ordinary skill in the art that some of these chemicals are incompatible at certain combinations, and accordingly, are easily substituted with different chemicals that have similar properties but are compatible in the relevant mixture.

**Table 8**

| **Sr. No.** | **Ingredient** | **Concentration** |
|---|---|---|
| 1. | TNFR:Fc | 50.0 mg/ml |
| 2. | Sodium dihydrogen phosphate | 1.5 mg/ml |
| 3. | Dipotassium hydrogen phosphate | 3.3 mg/ml |
| 4. | Lysine | 1.5 mg/ml |
| 5. | Sodium chloride | 5.8 mg/ml |
| 6. | Polysorbate 20 | 0.1 mg/ml |
| 7. | Sucrose | 20 mg/ml |
| 8. | Aspartic acid | 1.3 mg/ml |
| 9. | Disodium EDTA | 0.37 mg/ml |

### a) Stability test of TNFR:Fc aqueous formulation

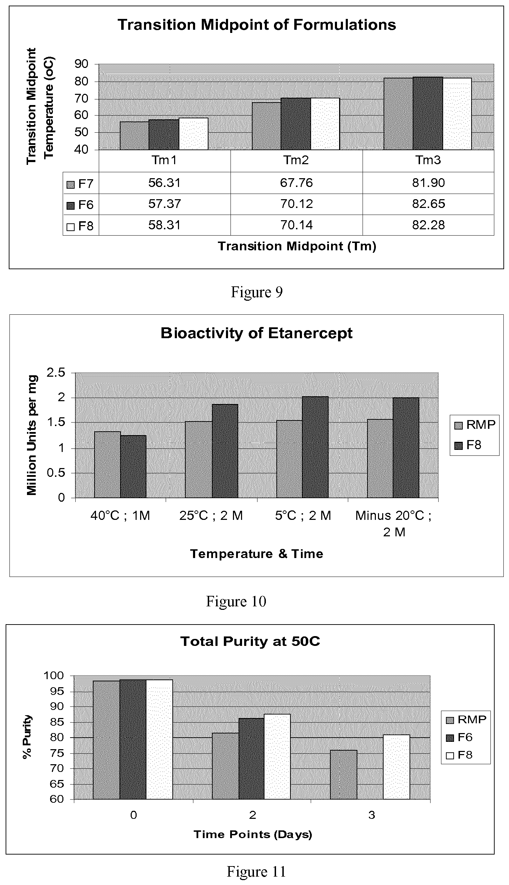

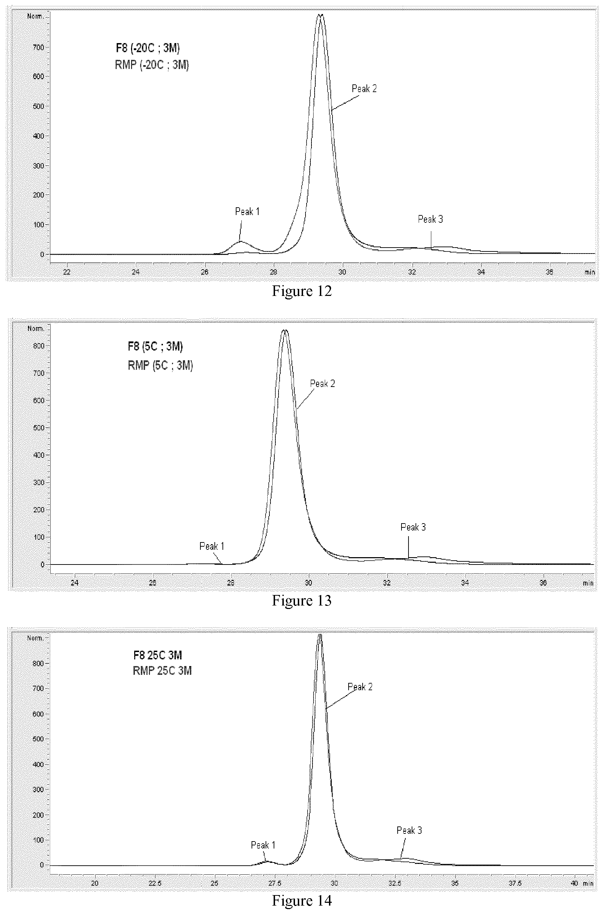

The biological tests were performed in compliance with the regulations of the European Pharmacopeia.
- **SDS PAGE [Aggregation (Non Reduced) and Fragmentation (Reduced)]**
   Due to high propensity to aggregation in case of high concentration monoclonal antibodies and fusion proteins, non-reducing SDS PAGE was utilized to assess covalent aggregates. As TNFR binds to hinge region of Fc by disulphide bonds and since there are many disulphide bonds present in Fc and TNFR, the fragmentation of proteins was checked under reducing SDS PAGE.
- **SE-HPLC (Aggregation and Fragmentation)**
   As size exclusion chromatography separates proteins and its related impurities on the basis of their size the above technique was useful to detect aggregation and fragmentation of Etanercept.
- **HIC (Aggregation, fragmentation, Truncation and Misfolding)**
   HIC separates proteins on the basis of hydrophobicity. Peak 1 in HIC determines fragments, Peak 2 corresponds to active dimer of Etanercept and Peak 3 corresponds to aggregates. Pre-peak 1 observed in some cases corresponds to truncated impurities. Reference profiles of HIC defining Peak 1, Peak 2 and Peak 3 are given in Figure 12 (-20°C after 3 months), Figure 13 (5±3°C after 3 months), Figure 14 (25±2°C after 3 months).
- **Differential Scanning Calorimetry (DSC)**
   DSC is a technique used to determine thermodynamic stability of protein. Etanercept has three transitions and Figure 9 is indicative that Tm1 corresponds to TNFR, Tm2 corresponds to CH2 domain and Tm3 corresponds to CH3 domain of Fc portion (Higher the Tm determines higher stability).
- ***In vitro* Bioassay (Bioactivity)**
   The method is based on principle of neutralization of cytotoxic effect. TNF-α produces cytotoxic effect on L929 (mouse connective tissue) cell line. TNFR: Fc specifically neutralizes the cytotoxic activity of TNF- α in a dose dependent manner (Figure 10). The bioactivity of RMP is 1.7 Million units per mg.

The aspects with respect to stability of aqueous formulation are captured in Table 9.

**Table 9 - Analytical parameters for a liquid formulation of Etanercept at zero time and after storage at 5°C, 25°C and -20°C for 1, 2 and 3 months**

| **Assay** | **Zero Time** | | | **1 month** | | | **2 months** | | | **3 months** | | |
|---|---|---|---|---|---|---|---|---|---|---|---|---|
| | -20°C | 5±3°C | 25±2° C | -20°C | 5±3° C | 25±2 °C | -20°C | 5±3°C | 25±2°C | -20°C | 5±3°C | 25±2° C |
| Aggregation by SE-HPLC | 1.4% | 1.4 % | 1.4 % | 1.7 % | 1.2% | 1.4% | 1.8% | 1.1 % | 1.7 % | 1.7 % | 1.1 % | 1.9 % |
| Fragmentation by SE-HPLC | 0% | 0% | 0 % | 0.1 % | 0.1 % | 0.2 % | 0.05 % | 0.2 % | 0.4 % | 0.0 % | 0.1 % | 0.5 % |
| HIC Peak 1 | 0 % | 0 % | 0 % | Not tested | Not tested | Not tested | 0.21 % | 0.28 % | 0.90% | 0.81 % | 0.5 % | 1.4 % |
| HIC Peak 2 | 93.7 % | 93.7 % | 93.7 % | Not tested | Not tested | Not tested | 93.1% | 94.1 % | 92.8 % | 93.8 % | 93.6 % | 92.9 % |
| HIC Peak 3 | 6.3 % | 6.3 % | 6.3 % | Not tested | Not tested | Not tested | 6.7 % | 5.7 % | 6.3 % | 5.4 % | 5.9% | 5.7% |

### Example 6

### Stability test of TNFR: Fc liquid formulations at 40°C

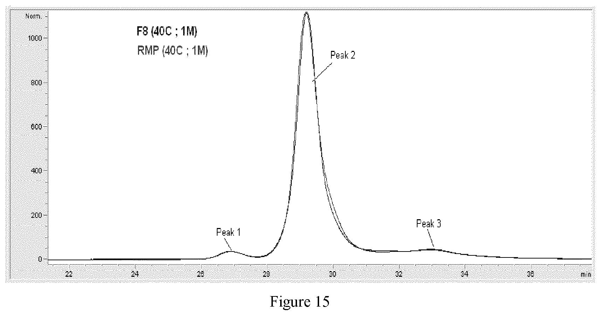

Liquid formulation comprising Etanercept having the composition given in Table 8 was prepared. Formulated solution was sterilized by using 0.2 µm filter under laminar air flow and stored at 40 °C for 1 month. The formulation was analyzed by SE-HPLC, SDS-PAGE, and HIC (Figure 15) at zero time, 7 days, 15 days, 21 days and 1 month of storage at 40°C and bioactivity was also checked. The results are reported in Table 10.

**Table 10: Results of stability tests for Etanercept formulation**

| **Assay** | **Zero Time** | **7 days** | **15 days** | **21 days** | **1 month** |
|---|---|---|---|---|---|
| | **40°C** | **40°C** | **40°C** | **40°C** | **40°C** |
| Aggregation by SE-HPLC | 1.4 % | 1.9 % | 2.4 % | 3.7% | 5.7 % |
| Fragmentation by SE-HPLC | 0% | 0.3 % | 0.5 % | 0.6 % | 1.1 % |
| HIC Peak 1 | 0% | Not tested | Not tested | Not tested | 2.7 % |
| HIC Peak 2 | 93.7 % | Not tested | Not tested | Not tested | 90.0 % |
| HIC Peak 3 | 6.3 % | Not tested | Not tested | Not tested | 7.2 % |

### Example 7

### Stability test of TNFR: Fc liquid formulations at 50°C

Liquid formulation comprising Etanercept having the composition given in Table 8 was prepared. Formulated solution was sterilized by using 0.2 µm filter under laminar air flow and stored at 50°C for 3 days. The formulation was analyzed by SE-HPLC, SDS-PAGE, and HIC at zero time, and after 2 days and 3 days of storage at 50°C. The results are reported in Table 11.

**Table 11: Results of stability tests for Etanercept formulation (50 °C)**

| **Assay** | **Zero Time** | **2 days** | **3 days** |
|---|---|---|---|
| | **50°C** | **50°C** | **50°C** |
| Aggregation by SE-HPLC | 1.4 % | 12.5 % | 19.1 % |
| Fragmentation by SE-HPLC | 0% | 0% | 0% |
| HIC Peak 1 | 0% | 0% | 0% |
| HIC Peak 2 | 93.7 % | 92.6 % | 84.9 % |
| HIC Peak 3 | 6.3 % | 7.4 % | 15.1% |

The novel formulations of fusion protein are prepared using suitable combination of buffer, an aggregation inhibitors, tonicity modifiers, stabilizers, surfactants, chelating agents and optionally with preservative in suitable combinations thereof.

The formulation prepared by the said invention comprises an effective amount of biologically active TNFR: Fc which is used in treating inflammatory diseases in humans. They are preferably used as injectable aqueous solutions.

## Claims

1. An aqueous pharmaceutical formulation comprising TNFR:Fc, an aggregation inhibitor, a buffer, a non-ionic surfactant, a stabilizer, a tonicity modifier, and optionally a preservative, wherein the aggregation inhibitor is aspartic acid, optionally in combination with lysine and wherein the pharmaceutical formulation further comprises EDTA as chelating agent and fragmentation inhibitor.

2. The pharmaceutical formulation of claim 1, wherein the buffer comprises phosphate buffer, bicarbonate buffer, succinate buffer, acetate buffer, citrate buffer, amino acids, TRIS buffer, either alone or in suitable combination, giving desired pH range from 5.5 to 7.5, and preferably the buffer is selected from the group consisting of sodium phosphate, histidine, potassium phosphate, sodium or potassium citrate, maleic acid, ammonium acetate, tris-(hydroxymethyl)- aminomethane (tris), acetate and diethanolamine or a combination thereof, and more preferably the buffer is phosphate buffer in combination with its sodium and potassium salts.

3. The pharmaceutical formulation of any of claims 1 and 2, wherein the non-ionic surfactant is selected from the group consisting of a polysorbate-based non-ionic surfactant and a poloxamer-based non-ionic surfactant or a combination thereof, and preferably the non-ionic surfactant is polysorbate 20.

4. The pharmaceutical formulation of any of claims 1 to 3, wherein the stabilizer is selected from the group consisting of amino acids such as glycine, alanine, lysine, proline and serine and their salts thereof, either alone or in combination, monosaccharides such as glucose and mannose, and their likes, either alone or in combination, disaccharides such as sucrose, trehalose, and maltose, and their likes, either alone or in combination, sugar alcohols such as mannitol and sorbitol, and their likes, either alone or in combination, and polysaccharides such as dextran, polyethylene glycol and their likes, either alone or in combination, and preferably the stabilizer is sucrose.

5. The pharmaceutical formulation of any of claims 1 to 4, wherein the tonicity modifier is selected from the group consisting of sodium chloride, potassium chloride, sodium sulfate or a combination thereof, and preferably the tonicity modifier is sodium chloride.

6. The pharmaceutical formulation of any of claims 1 to 5, wherein the preservative is selected from the group consisting of phenol, m-cresol, p-cresol, o-cresol, chloro-cresol, alkylparaben (methyl, ethyl, propyl, butyl and the like), benzethonium chloride, sodium dehydroacetate or thiomersal, either alone or in combination thereof

7. The pharmaceutical formulation of any of claims 1 to 6 comprising aspartic acid and lysine as aggregation inhibitors, sodium-potassium phosphate buffer giving desired pH range from 5.5 to 7.5, sodium chloride as tonicity modifier, sucrose as stabilizer, polysorbate 20 as a non-ionic surfactant, and EDTA as fragmentation inhibitor and chelating agent, wherein TNFR:Fc is present at a concentration of 10 mg/ml to 100 mg/ml.

8. The formulation of claim 7, comprising from 25 to 50 mg/ml of TNFR:Fc, from 1 mM to 50 mM of aspartic acid, from 1 mM to 50 mM of lysine, from 10 mM to 50 mM of sodium-potassium phosphate, from 0.75 % to 3 % of sucrose, from 50 to 150 mM of NaCl, from 0.1 mM to 2 mM of EDTA, from 0.05 mg/ml to 1.0 mg/ml of polysorbate 20, and at pH 6.0 to pH 7.0.

9. The formulation of any of claims 1 to 8, which is an injectable aqueous solution.

10. Use of EDTA for preventing fragmentation of TNFR:Fc in an aqueous pharmaceutical formulation.

## Patentansprüche

1. Wässrige pharmazeutische Formulierung, umfassend TNFR:Fc, einen Aggregationsinhibitor, einen Puffer, ein nichtionisches oberflächenaktives Mittel, einen Stabilisator, einen Tonizitätsmodifikator und gegebenenfalls ein Konservierungsmittel, wobei der Aggregationsinhibitor Asparaginsäure ist, gegebenenfalls in Kombination mit Lysin, und wobei die pharmazeutische Formulierung zudem EDTA als Chelatbildner und Fragmentierungsinhibitor umfasst.

2. Pharmazeutische Formulierung nach Anspruch 1, wobei der Puffer Phosphatpuffer, Bicarbonatpuffer, Succinatpuffer, Acetatpuffer, Citratpuffer, Aminosäuren, TRIS-Puffer, entweder allein oder in einer geeigneten Kombination, umfasst, so dass ein gewünschter pH-Wert-Bereich von 5.5 bis 7.5 erhalten wird, und wobei der Puffer vorzugsweise ausgewählt ist aus der Gruppe, bestehend aus Natriumphosphat, Histidin, Kaliumphosphat, Natrium- oder Kaliumcitrat, Maleinsäure, Ammoniumacetat, Tris(hydroxymethyl)aminomethan (Tris), Acetat und Diethanolamin oder einer Kombination davon, und wobei der Puffer stärker bevorzugt Phosphatpuffer in Kombination mit seinen Natrium- und Kaliumsalzen ist.

3. Pharmazeutische Formulierung nach einem der Ansprüche 1 und 2, wobei das nichtionische oberflächenaktive Mittel ausgewählt ist aus der Gruppe, bestehend aus einem nichtionischen oberflächenaktiven Mittel auf Polysorbat-Basis und einem nichtionischen oberflächenaktiven Mittel auf Poloxamer-Basis oder einer Kombination davon und wobei das nicht-ionische oberflächenaktive Mittel vorzugsweise Polysorbat 20 ist.

4. Pharmazeutische Formulierung nach einem der Ansprüche 1 bis 3, wobei der Stabilisator ausgewählt ist aus der Gruppe bestehend aus Aminosäuren wie Glycin, Alanin, Lysin, Prolin und Serin und deren Salzen, entweder allein oder in Kombination, Monosacchariden, wie Glucose und Mannose und dergleichen, entweder allein oder in Kombination, Disacchariden, wie Saccharose, Trehalose und Maltose und dergleichen, entweder allein oder in Kombination, Zuckeralkoholen, wie Mannit und Sorbit und dergleichen, entweder allein oder in Kombination, und Polysacchariden, wie Dextran, Polyethylenglycol und dergleichen, entweder allein oder in Kombination und wobei der Stabilisator vorzugsweise Saccharose ist.

5. Pharmazeutische Formulierung nach einem der Ansprüche 1 bis 4, wobei der Tonizitätsmodifikator ausgewählt ist aus der Gruppe bestehend aus Natriumchlorid, Kaliumchlorid, Natriumsulfat oder einer Kombination davon, wobei der Tonizitätsmodifikator vorzugsweise Natriumchlorid ist.

6. Pharmazeutische Formulierung nach einem der Ansprüche 1 bis 5, wobei das Konservierungsmittel ausgewählt ist aus der Gruppe, bestehend aus Phenol, m-Kresol, p-Kresol, o-Kresol, Chlorkresol, Alkylparaben (Methyl, Ethyl, Propyl, Butyl und dergleichen), Benzethoniumchlorid, Natriumdehydroacetat oder Thiomersal, entweder allein oder in Kombination davon.

7. Pharmazeutische Formulierung nach einem der Ansprüche 1 bis 6, umfassend Asparaginsäure und Lysin als Aggregationsinhibitoren, Natrium-Kaliumphosphat-Puffer, der den gewünschten pH-Wert-Bereich von 5.5 bis 7.5 ergibt, Natriumchlorid als Tonizitätsmodifikator, Saccharose als Stabilisator, Polysorbat 20 als nichtionisches oberflächenaktives Mittel und EDTA als Fragmentierungsinhibitor und Chelatbildner, wobei TNFR:Fc in einer Konzentration von 10 mg/ml bis 100 mg/ml zugegen ist.

8. Formulierung nach Anspruch 7, umfassend 25 bis 50 mg/ml TNFR:Fc, 1 mM bis 50 mM Asparaginsäure, 1 mM bis 50 mM Lysin, 10 mM bis 50 mM Natrium-KaliumPhosphat, 0.75% bis 3% Saccharose, 50 bis 150 mM NaCl, 0.1 mM bis 2 mM EDTA, 0.05 mg/ml bis 1.0 mg/ml Polysorbat 20 und bei einem pH-Wert von 6.0 bis zu einem pH-Wert von 7.0.

9. Formulierung nach einem der Ansprüche 1 bis 8, bei der es sich um eine injizierbare wässrige Lösung handelt.

10. Verwendung von EDTA zur Verhinderung der Fragmentierung von TNFR:Fc in einer wässrigen pharmazeutischen Formulierung.

## Revendications

1. Formule pharmaceutique aqueuse comprenant TNFR:Fc, un inhibiteur d'agrégation, un tampon, un tensioactif non ionique, un stabilisant, un modificateur de tonicité, et éventuellement un conservateur, où l'inhibiteur d'agrégation est l'acide aspartique, éventuellement en combinaison avec de la lysine et où la formule pharmaceutique comprend en outre de l'EDTA en tant qu'agent chélatant et un inhibiteur de fragmentation.

2. Formule pharmaceutique selon la revendication 1, où le tampon est constitué de tampon phosphate, de tampon bicarbonate, de tampon succinate, de tampon acétate, de tampon citrate, d'acides aminés, de tampon TRIS, seuls ou dans une combinaison adaptée, pour obtenir un intervalle de pH souhaité entre 5.5 et 7.5, et préférentiellement, le tampon est choisi dans le groupe constitué par phosphate de sodium, histidine, phosphate de potassium, citrate de sodium ou de potassium, acide maléique, acétate d'ammonium, tris-(hydroxyméthyl)-aminométhane (tris), acétate et diéthanolamine ou l'une de leurs combinaisons, et plus préférentiellement, le tampon est un tampon phosphate en combinaison avec ses sels de sodium et de potassium.

3. Formule pharmaceutique selon l'une quelconque des revendications 1 et 2, où le tensioactif non ionique est choisi dans le groupe constitué par un tensioactif non ionique à base de polysorbate et un tensioactif non ionique à base de poloxamère, ou l'une de leurs combinaisons, et préférentiellement, le tensioactif non ionique est le polysorbate 20.

4. Formule pharmaceutique selon l'une quelconque des revendications 1 à 3, où le stabilisant est choisi dans le groupe constitué par les acides aminés tels que glycine, alanine, lysine, proline et sérine et leurs sels, seuls ou combinés, les monosaccharides tels que glucose et mannose, et similaires, seuls ou combinés, les disaccharides tels que sucrose, tréhalose et maltose et similaires, seuls ou combinés, les alcools sucrés tels que mannitol et sorbitol et similaires, seuls ou combinés, et les polysaccharides tels que dextrane, polyéthylène glycol et similaires, seuls ou combinés, et préférentiellement, le stabilisant est le sucrose.

5. Formule pharmaceutique selon l'une quelconque des revendications 1 à 4, où le modificateur de tonicité est choisi dans le groupe constitué par chlorure de sodium, chlorure de potassium, sulfate de sodium ou l'une de leurs combinaisons, et préférentiellement, le modificateur de tonicité est le chlorure de sodium.

6. Formule pharmaceutique selon l'une quelconque des revendications 1 à 5, où le conservateur est choisi dans le groupe constitué par phénol, m-crésol, p-crésol, o-crésol, chloro-crésol, alkylparabène (méthyle, éthyle, propyle, butyle, etc.), chlorure de benzéthonium, déshydroacétate de sodium ou thiomersal, seuls ou combinés.

7. Formule pharmaceutique selon l'une quelconque des revendications 1 à 6, comprenant de l'acide aspartique et de la lysine en tant qu'inhibiteurs d'agrégation, un tampon phosphate de sodium-potassium pour obtenir l'intervalle de pH souhaité entre 5.5 et 7.5, du chlorure de sodium en tant que modificateur de tonicité, du sucrose en tant que stabilisant, du polysorbate 20 en tant que tensioactif non ionique, et de l'EDTA en tant qu'inhibiteur de fragmentation et agent chélatant, où TNFR:Fc est présent à une concentration comprise entre 10 mg/ml et 100 mg/ml.

8. Formule selon la revendication 7, comprenant entre 25 et 50 mg/ml de TNFR:Fc, entre 1 mM et 50 mM d'acide aspartique, entre 1 mM et 50 mM de lysine, entre 10 mM et 50 mM de phosphate de sodium et de potassium, entre 0.75 % et 3 % de sucrose, entre 50 et 150 mM de NaCl, entre 0.1 mM et 2 mM d'EDTA, entre 0.05 mg/ml et 1.0 mg/ml de polysorbate 20, et à un pH compris entre 6.0 et 7.0.

9. Formule selon l'une quelconque des revendications 1 à 8, qui est une solution aqueuse injectable.

10. Utilisation d'EDTA dans la prévention de la fragmentation de TNFR:Fc dans une formule pharmaceutique aqueuse.
